Europäisches Patentamt

European Patent Office

Office européen des brevets

(11) Publication number: **0 640 347 A1**

## EUROPEAN PATENT APPLICATION
### published in accordance with Art. 158(3) EPC

(21) Application number: 93904375.8

(22) Date of filing: 02.03.93

(86) International application number: **PCT/JP93/00264**

(87) International publication number: **WO 93/17702 (16.09.93 93/22)**

(51) Int. Cl.⁶: **A61K 39/00**

(30) Priority: **03.03.92 JP 45528/92**

(43) Date of publication of application: **01.03.95 Bulletin 95/09**

(84) Designated Contracting States: **AT BE CH DE DK ES FR GB GR IE IT LI NL PT SE**

(71) Applicant: **DAIICHI PHARMACEUTICAL CO., LTD.**
**14-10, Nihonbashi 3-chome**
**Chuo-ku,**
**Tokyo 103 (JP)**

(72) Inventor: **TSUCHIYA, Seishi**
**29-12, Higashitoyoda 1-chome, Hino-shi**
**Tokyo 191 (JP)**
Inventor: **ARAMAKI, Yukihiko**
**102, Park Heiz,**
**517-24, Kitano-cho**
**Hachioji-shi,**
**Tokyo 192 (JP)**
Inventor: **HARA, Toshifumi, 203, Tokyo College**
**of Pharmacy Shokuinryo,**
**2258-8, Mejirodai 2-chome**
**Hachioji-shi,**
**Tokyo 193 (JP)**
Inventor: **KIKUCHI, Hiroshi, Daiichi Pharmaceutical Co., Ltd.**
**Tokyo R&D Center,**
**16-13, Kitakasai 1-chome**
**Edogawa-ku,**
**Tokyo 134 (JP)**
Inventor: **YACHI, Kiyoto, Daiichi Pharmaceutical Co., Ltd.**
**Tokyo R&D Center,**
**16-13, Kitakasai 1-chome**
**Edogawa-ku,**
**Tokyo 134 (JP)**
Inventor: **IKEUCHI, Tohru, Daiichi Pharmaceutical Co., Ltd.**
**Tokyo R&D Center,**
**16-13, Kitakasai 1-chome**
**Edogawa-ku,**
**Tokyo 134 (JP)**

(74) Representative: **Vossius, Volker, Dr.**
**Dr. Volker Vossius**
**Patentanwaltskanzlei - Rechtsanwaltskanzlei**
**Holbeinstrasse 5**
**D-81679 München (DE)**

(54) **ORAL VACCINE.**

(57) An oral vaccine comprising a complex of an antigen with a lipid, wherein the lipid contains a phospholipid comprising a glycolipid and/or phosphatidylserine combined with mannose. The vaccine serves to produce efficiently an antibody against the above antigen after being orally administered to living organisms. It makes it possible to administer orally a microbial antigen or a weakly virulent microbe which has not been orally absorbable and has been incapable of producing an antibody thereagainst.

Technical Field of the Invention

The present invention relates to an oral vaccine capable of efficiently producing antibodies after the oral administration. The oral vaccine of the present invention efficiently produces antibodies to antigens kept therein after the oral administration thereof to exhibit an effect of prevention from infection.

Background art

Most vaccines currently used are in the form of a subcutanesous injection. However, the subcutaneous injection has clinical problems such as infrequent side effects and a pain caused by the injection. Various investigations are made on the administration method for the purpose of solving the problems. The development of an oral vaccine is the most desirable, since the dosage form thereof is safe and the administration thereof is convenient.

Although investigations have been hitherto made on the oral vaccine with a buffering agent, tablets with enteric coating, microparticles, nano-particles and adjuvants, they have not been succeeded yet [International Journal of Pharmaceutics, 75, 1 (1991); and Journal of Clinical Pharmacy and Therapeutics, 16, 309 (1991)]. In the general remarks of them, some examples of the application of liposomes having an orthodox membrane composition to the oral vaccine are reported. However, these techniques are far from the practical application, while the antibody titer is elevated to some extent.

Thus, no means of efficiently producing the antibodies after the oral administration have been developed yet in the prior art.

It is known to administer liposomes having a membrane modified with phosphatidylserine by intravenous injection [Cancer Research, 40, 4460 (1980)], and it was reported that liposomes having a membrane modified with a mannose derivative or mannan derivative are cumulated in the liver and lungs [Biochimica et Biophysica Acta, 632, 562 (1980), and "Byotai Seiri (Pathophysiology)", 6, 771 (1985)].

Disclosure of the Invention

The object of the present invention is to provide an oral vaccine capable of efficiently producing antibodies to microbial antigens kept therein or antigens comprising attenuated microorganisms after the oral administration thereof to the living body.

The present invention relates to an oral vaccine comprising a complex of antigens and lipids. The antibody titer of the complex can be elevated even by the oral administration, since it comprises a glycolipid having mannose as a lipid and/or a phospholipid which is phosphatidylserine.

Complexes can be classified into (a) aggregates comprising irregularly arranged protein or glycoprotein as the antigens and the lipid through a hydrophobic bond and/or hydrogen bond and (b) lipid membrane structure.

The lipid membrane structure of the present invention is to be understood in a broad sense. In some cases, the lipid forms a membrane and, in other cases, a membrane is formed between the oil component and the aqueous component. In other words, they can be said to be particles in which polar groups of an amphipathic lipid are arranged at the interface toward the aqueous phase. Examples of them include liposomes, emulsions and water-soluble micelles.

The present invention has been completed on the basis of a finding that the above-described problems can be solved by the fact that the complex comprises a glycolipid having mannose as the lipid and/or a phospholipid which is phosphatidylserine.

Best Mode for Carying Out the Invention

The vaccine of the present invention may further contain an adjuvant, if necessary.

The antigens used for forming the vaccines are well known in the art. The microbial antigens and attenuated microorganisms include protein antigens of microorganisms, for example, viruses such as influenza virus A, influenza virus B, influenza virus C, rotavirus, cytomegalovirus, RS virus, adenovirus, AIDS virus (HIV), hepatitis virus C, hepatitis virus A, hepatitis virus B, varicella zoster virus, herpes simplex virus 1 and 2, ATL (adult T cell leukemia) virus, Coxsackie virus, enterovirus, exanthema subitum virus (HHV-6), measles virus, rubella virus, mumps virus, poliovirus, Japanese encephalitis virus and rabies virus; fungi such as cariogenic streptococcus, cholera vibrio, hemophilus influenzae, Streptocuccus pneumoniae, Bordetella pertussis, Corynebacterium diphtheriae and Clostridium tetanii; rickettsiae such as chlamydia; and protozoa such as plasmodium malariae; as well as attenuated microorganisms derived from them.

Where the microbial protein antigens are influenza viruses, examples of the oral vaccine of the present invention include the vaccine carrying hemagglutinin (HA), neuraminidase (NA) or a mixture thereof.

These antigens can be obtained by processing microorganisms and then purifying the product, by synthesis or genetic engineering.

The antigens which can be carried by the oral vaccine of the present invention vary depending on the kind of the lipid membrane structure. For example, the antigens which can be carried by liposomes are not particularly limited and they include, for example, water-soluble or fat-soluble microbial antigens and attenuated microorganisms. The antigens which can be carried by emulsions include fat-soluble antigens and those which can be carried by the micelles are water-soluble antigens.

The phosphatidylserines in the present invention are those derived from natural products such as soybeans and egg yolks, hydrogenated phosphatidylserines produced by hydrogenating these phosphatidylserines, phosphatidylserines semi-synthesized from the natural products such as soybeans and egg yolks, hydrogenated phosphatidylserines produced by hydrogenating the semi-synthesized ones, and synthesized or semi-synthesized dimyristoylphosphatidylserines, dipalmitoylphosphatidylserines and distearoylphosphatidylserines. Among them, preferred are hydrogenated phosphatidylserines, dipalmitoylphosphatidylserines and distearoylphosphatidylserines.

The glycolipids having mannose in the present invention include, for example, mannose residue-containing phosphatidylethanolamine derivatives [Biocheimica et Biophysica Acta, 632, 562 (1980)], cholesterol derivatives [Proc, Natl. Acad. Sci., U.S.A,, 77, 4430 (1980)], dimannosyl diglyceride [Biochemical and Biophysical Research Communications, 110, 140 (1983)], mannobiose fatty acid esters and amides [Japanese Patent Unexamined Published Application (hereinafter referred to as "J.P. KOKAI") No. Hei 1-104088], and phosphatidyl mannoses which are derivatives obtained by partially substituting mannan (polysaccharide) with a fatty acid or cholesterol [Byotai Seiri (Pathophysiology) 6, 771 (1985)]. More specifically, they include 4-O-(6-0-eicosanoyl-$\beta$-D-mannopyranosyl)-D-mannopyranose and N-(2-N'-cholesterylcarboxymethyl)carbamylmethylmannan.

The phospholipids (phosphatidylserines) and glycolipids used as the membrane component are used either singly or in the form of a mixture of them. They are added to the lipid membrane structure mainly comprising a phosphatidylcholine (lecithin), sphingomyelin or other membrane component or, alternatively, they can be used singly for forming the lipid membrane structure without using such a membrane component.

Namely, the phosphatidylserines are capable of forming the liposomes and emulsion without using any other component, and the glycolipids having mannose (mannobiose fatty acid esters and amides) are capable of forming the emulsions or micelles without using other components. Therefore, in the present invention, the upper limit of the phosphatidylserine or glycolipid having mannose used as the membrane component for the lipid membrane structure is not limited at all. The lower limit thereof may be 1 molar % or more, preferably 5 to 50 molar %, based on the whole lipid membrane components.

Other membrane components include phosphatidylcholines (lecithin) such as phosphatidylcholine per se, hydrogenated phosphatidylcholines produced by hydrogenating phosphatidylcholines derived from natural products such as soybeans and egg yolks, synthesized or semi-synthesized dimyristoylphosphatidylcholines, synthesized or semi-synthesized dipalmitoylphosphatidylcholine, synthesized or semi-synthesized distearoylphosphatidylcholines and sphingomyelin.

It is also possible to physically stabilize the liposomes by adding other charged lipids such as dialkyl phosphates, diacyl phosphatidic acids, stearylamines, phosphatidylglycerols, phosphatidylinositols, phosphatidylethanolamines and ionic surfactants (acidic or basic).

Sterols such as cholesterol can be added to the liposomes in order to physically and biologically stabilizing them.

An adjuvant can be further added thereto, if nececcesary, in the present invention. Examples of the adjuvants include (N-acetylmuramoyl)-L-alanyl-D-isoglutamine (muramyl dipeptide, MDP) which is the minimum effective adjuvant contained in the cell wall of Mycobacterium tuberculosis bacillus and derivatives thereof such as 6-O-(2-tetradecylhexadecanoyl)-N-acetylmuramoyl)-L-alanyl-D-isoglutamine (B30-MDP), 6-O-(2-tetradecylhexadecanoyl)-N-acetylmuramoyl)-L-alanyl-D-gluramamide (B30-MDP amide) and $N^2$-[(N-acetylmuramoyl)-L-alanyl-D-isoglutaminyl]-$N^6$-stearoyl-L-lysine (MDP-Lys[L18]). When the water-soluble muramyl dipeptide is used, the lipid membrane structure can be the liposomes, and it can be kept in the inner aqueous phase of the liposomes. The amount of the muramyl dipeptide is not particularly limited, and it can be added in an amount of up to the saturation point in the aqueous solvent. When the fat-soluble muramyl dipeptide derivative is used, the lipid membrane structure can be the liposomes, emulsions and micelles. Although the relative amount thereof to the whole lipid membrane components is not particularly limited, it is preferably at least 0.1 molar %, more preferably 1 to 30 molar %.

The vaccine of the present invention is characterized in that the antibody titer can be elevated to prevent the infection when it is orally administered. The dose of, for example, influenza vaccine is 50 $\mu$ g to 5 mg, usually 200$\mu$ g to 2 mg per adult. The vaccine in such a dose can be administered once, or twice at an interval of a week, two weeks, three weeks or one month. Also, it can be administered three, four, five or six times at such an interval. Usually, it is administered once or twice to four times at an interval of one month. The dose of hepatitis virus B is 20 $\mu$ g to 5 mg, usually 50 $\mu$ g to 2 mg per adult person. It is usable in the same manner as that of a vaccine to be injected. The dose of a vaccine for other antigens is 1 to 20 times as much as the vaccine to be injected. The dosage is thus suitably determined. As a matter of course, the dose is suitably changed depending on the immunological response, age, etc. of the individuals.

The toxicity of the phosphatidylcholines, phosphatidylserines and cholesterol used in the present invention are considered to be low, since they are components of the living bodies. Y. M. Rustum et al. tested the acute toxicity of liposomes prepared from the phosphatidylcholines, phosphatidylserines and cholesterol derived from egg yolks to find that the 50 % lethal dose of them was 5.5 g/kg or larger [refer to Cancer Research 39, 1390 to 1395 (1979)].

Then, the description will be made on the processes for producing the vaccines of the present invention.

a) Process for producing liposome-type vaccine:

An aqueous dispersion of liposomes is prepared from microbial antigens or an attenuated microorganism per se, a membrane-forming substance such as lecithin or sphingomyelin, and a phosphatidylserine and/or glycolipid having mannose and, if necessary, an adjuvant by a known method [for example, Annual Review of Biophysics and Bioengineering, 9, 467 (1980)]. The liposomes may further contain a stabilizer such as a sterol, e.g. cholesterol, charged lipid, e.g. a dialkyl phosphate, diacylphosphatidic acid or stearylamine, or an antioxidant, e.g.$\alpha$ -tocopherol.

b) Process for producing emulsion-type vaccine:

An emulsion is prepared from microbial antigens or an attenuated microorganism per se, an amphipathic substance such as lecithin or a polyoxyethylene sorbitan fatty acid ester (Tween), an oil or fat such as soybean oil, phosphtidyl serine and/or glycolipid having mannose and, if necessary, an adjuvant by a known emulsion production method.

c) Process for producing micelle-type vaccine:

An aqueous micelle dispersion is prepared from microbial antigens or an attenuated microorganism per se, a micelle-forming surfactant (which is to be added to an aqueous solvent, in at least a critical concentration of micelle formation) such as a polyoxyethylene sorbitan fatty acid ester (Tween), a sodium salt of fatty acid or polyoxyethylene-hardened castor oil, phosphtidylserine and/or glycolipid having mannose and, if necessary, an adjuvant by a known micelle production method.

The oral vaccine of the present invention is capable of efficiently forming antibodies to the antigens kept therein after the oral administration to a living body. Therefore, the present invention makes it possible to produce the oral vaccine of microbial antigens or an attenuated microorganism, while such a vaccine could not be orally absorbed and the antibodies to the antigens could not be produced in the prior art.

Examples

The following Examples and Test Examples will further illustrate the present invention, by using bovine serum albumin (BSA), influenze virus antigens and hepatitis virus B as the models of microbial protein antigens, which by no means limit the invention. In the following Examples, an antigen protein is occasionally present apart from the lipid membrane structure without being included therein or a complex in another form is occasionally formed, but it can be removed by ultracentrifugation, gel filtration or the like or the product can be used as it is without removing it.

Examples 1 to 4

Liposome-type oral vaccin containning bovine serum albumin (BSA):

a) Preparation of liposomes containing BSA sealed therein:

20 ml of liquid chloroform / methanol (9/1) mixture was added to 160 mg of a lipid comprising distearoylphosphatidylcholine (DSPC) / hydrogenated phosphatidylserine (H-PS) / cholesterol (Chol) in a molar ratio of 1/1/2 to dissolve the lipid into the liquid mixture. The solvent was distilled off by heating at 37 °C with a rotary evaporator. After drying in vacuo for two hours, a lipid film was obtained. 4 ml of each of solutions of various concentrations of BSA (see Table 1) in physiological saline was added to the lipid film, and the resultant mixture was stirred in a vortex mixer at 50°C for 10 min to obtain multilamellar liposomes (MLV; multilamellar vesicles). The resultant product was then centrifuged (35,000 x g, 4°C , 20 minutes) to remove the supernatant liquid. The precipitate was suspended again in 4 ml of physiological saline. The centrifugation process was repeated three times to remove free BSA. The liposome suspension thus prepared was dialyzed with 3 ℓ of physiological saline through a $0.1\mu$ m polycarbonate membrane filter for 12 hours to remove liposomes having a diameter of 0.1 $\mu$ m or below, thereby finally obtaining a liposome-type oral vaccine containing BSA sealed therein as shown in the right column in Table 1.

Table 1

| Formulation of liposomes | | | | |
|---|---|---|---|---|
| Ex. | Amount of lipid (mg) | BSA Conc. (mg/ml) | Final liposome suspension | |
| | | | Lipid conc. ($\mu$ mol/mol) | BSA conc. ($\mu$ g/ml) |
| 1 | 160 | 1.5 | 20 | 100 |
| 2 | 160 | 3.0 | 20 | 200 |
| 3 | 160 | 7.5 | 20 | 400 |
| 4 | 160 | 15.0 | 20 | 800 |
| Final liposome suspension: DSPC/H-PS/Chol = 54.4/51.2/54.4 (mg) | | | | |

Since the dose of the product for each mouse was 0.5 ml, the amount of the lipid given to the mouse was $10\mu$ mol and that of BSA was in the range of $50\mu$ g (Example 1) to $400\mu$ g (Example 4).

b) Immunity test:

The BSA-containing liposome suspension thus prepared or an aqueous solution of only BSA as a control (the dose of BSA was equal to that of the liposome group) was orally administered to each of BALB/c mice (male, 7-weeks old) (each group consisted of 6 mice) at a dose of 0.1 ml with a gastric probe according to a schedule given in Table 2. To determine the antibody titer of the blood and saliva, the blood was taken from the orbits and the saliva was taken by anesthetizing each mouse by intraperitoneal injection of 0.1 ml of 12 mg/ml pentobarbital, administering 0.2 mg/ml pilocarpine by the intraperitoneal injection and taking the saliva from the mouth with a Pasteur pipette.

Table 2

| Immunization schedule | | | | |
|---|---|---|---|---|
| Test | Immunization method | Day of oral administration | Day of taking blood | Day of taking saliva |
| 1 | Continuous administration for 5 days | 1, 2, 3, 4 and 5 | 6, 11, 18 and 26 | |
| 2 | 5 times at interval of 1 week | 1, 8, 15, 22 and 29 | 36, 46 and 55 | 55 |
| Dose of BSA in Test Example 1: 50, 100, 200 and $400\mu$ g/time/mouse | | | | |
| Dose of BSA in Test Example 2: 50, 100 and 400 $\mu$ g/time/mouse | | | | |

c) Determination of antibody titer:

IgG and IgA antibody titers in the serum and IgA antibody titer in the saliva were determined by ELISA method which will be described below.

[ELISA method]

$50\mu$ l of $100\mu$ g/ml BSA dissolved in a carbonic acid buffer solution (pH 9.4) was fed into each well of a microplate and left to stand at 4°C overnight to immobilize BSA as the antibodies on the microplate. It was washed three times each with $100\mu$ l/well of a phosphoric acid buffer solution (PBST) containing 0.05 % of Tween 20 to remove superfluous BSA. After blocking with 3 % skim mil, followed by washing with PBSDT three times to remove superfluous skim milk, $50\mu$ l/well of the serum of the mice two times, four times, eight times, sixteen times or the like diluted with PBST was added thereto. After the incubation at 25°C for 2 hours followed by washing with $100\mu$ l/well of PBST three times to remove the serum, $50\mu$ l of 500 ng/ml antibody IgG to mouse or IgA conjugate horseradish peroxidase was added to each well. After the incubation at 25°C for 2 hours followed by washing with $100\mu$ l/well of PBST three times to remove superfluous antibodies to mouse, $50\mu$ l/well of 500 ng/ml 2,2'-azino-bis(3-ethylbenzothiazoline-6-sulfonic acid) containing 0.015 % of hydrogen peroxide was added thereto. After the incubation at 37 °C for 20 minutes, the absorbance at 405 nm was determined with a microplate reader. The antibody titer was given in terms of $\log_2 2^n$ wherein 2n represents the maximum dilution rate which is above 0.2 for the serum and above 0.1 for the saliva.

d) Test results:

The IgG and IgA antibody titers in the serum and IgA antibody titer in the saliva determined by the test of the schedule given in Table 2 are given in Tables 3-1 to 3-4 and Tables 4-1 to 4-3, respectively.

In the continuous administration for 5 days in Test Example 1 (see Tables 3-1 to 3-4), the elevation of the antibody titer in the oral vaccine-given group was clearer than that in the control group wherein aqueous BSA solution was orally given, though IgG antibody titer in the serum was slightly elevated also in the control group. An increase of the IgA antibody titer in the serum was surely recognized in the oral vaccine-given group, while the IgA antibody titer was unchanged at all even when the dose was increased in the aqueous BSA solution-given group.

Table 3-1    Test Example 1 : Control Example 1 vs. Example 1

Dose of BSA = 50 $\mu$ g/mouse x 5 (day 1, 2, 3, 4 and 5)

| | IgG antibody titer in serum | | IgA antibody titer in serum | |
|---|---|---|---|---|
| | [Control Ex. 1] | [Ex. 1] | [Control Ex. 1] | [Ex. 1] |
| | Aqueous BSA solution | Liposome | Aqueous BSA solution | Liposome |
| day 1 | 1.0 ± 0.0 | 1.0 ± 0.0 | 1.0 ± 0.0 | 1.0 ± 0.0 |
| day 6 | 1.4 ± 0.6 | 3.1 ± 0.7 | undetermined | undetermined |
| day 11 | 1.3 ± 0.4 | 3.3 ± 1.3 | 1.0 ± 0.0 | 1.2 ± 0.2 |
| day 18 | 2.0 ± 1.0 | 4.5 ± 2.0 | 1.0 ± 0.0 | 2.0 ± 1.2 |
| day 26 | 1.7 ± 0.9 | 5.3 ± 1.7 | 1.0 ± 0.0 | 2.5 ± 1.5 |

(mean ± S.D.)

Table 3-2    Test Example 1 : Control Example 2 vs. Example 2

Dose of BSA = 100$\mu$ g/mouse x 5 (day 1, 2, 3, 4 and 5)

| | IgG antibody titer in serum | | IgA antibody titer in serum | |
|---|---|---|---|---|
| | [Control Ex. 2] | [Ex. 2] | [Control Ex. 2] | [Ex. 2] |
| | Aqueous BSA solution | Liposome | Aqueous BSA solution | Liposome |
| day 1 | 1.0 ± 0.0 | 1.0 ± 0.0 | 1.0 ± 0.0 | 1.0 ± 0.0 |
| day 6 | 1.7 ± 0.4 | 2.2 ± 0.4 | undetermined | undetermined |
| day 11 | 1.3 ± 0.3 | 4.2 ± 1.8 | 1.0 ± 0.0 | 1.0 ± 0.0 |
| day 18 | 1.5 ± 0.4 | 5.9 ± 2.1 | 1.0 ± 0.0 | 1.1 ± 0.1 |
| day 26 | 3.2 ± 0.8 | 6.9 ± 2.5 | 1.0 ± 0.0 | 3.5 ± 1.1 |

(mean ± S.D.)

7

Table 3-3  Test Example 1 : Control Example 3 vs. Example 3

Dose of BSA = 200μ g/mouse x 5 (day 1, 2, 3, 4 and 5)

| | IgG antibody titer in serum | | IgA antibody titer in serum | |
|---|---|---|---|---|
| | [Control Ex. 3] | [Ex. 3] | [Control Ex. 3] | [Ex. 3] |
| | Aqueous BSA solution | Liposome | Aqueous BSA solution | Liposome |
| day 1 | 1.0 ± 0.0 | 1.0 ± 0.0 | 1.0 ± 0.0 | 1.0 ± 0.0 |
| day 6 | 1.0 ± 0.0 | 3.0 ± 0.0 | undetermined | undetermined |
| day 11 | 1.5 ± 0.8 | 5.0 ± 1.2 | 1.0 ± 0.0 | 1.5 ± 0.5 |
| day 18 | 2.7 ± 1.8 | 7.5 ± 0.8 | 1.0 ± 0.0 | 3.3 ± 0.8 |
| day 26 | 3.0 ± 0.5 | 8.0 ± 1.2 | 1.0 ± 0.0 | 3.7 ± 1.6 |

(mean ± S.D.)

Table 3-4  Test Example 1 : Control Example 4 vs. Example 4

Dose of BSA = 400μ g/mouse x 5 (day 1, 2, 3, 4 and 5)

| | IgG antibody titer in serum | | IgA antibody titer in serum | |
|---|---|---|---|---|
| | [Control Ex. 4] | [Ex. 4] | [Control Ex. 4] | [Ex. 4] |
| | Aqueous BSA solution | Liposome | Aqueous BSA solution | Liposome |
| day 1 | 1.0 ± 0.0 | 1.0 ± 0.0 | 1.0 ± 0.0 | 1.0 ± 0.0 |
| day 6 | 1.5 ± 0.5 | 3.3 ± 0.4 | undetermined | undetermined |
| day 11 | 1.5 ± 0.5 | 5.0 ± 2.5 | 1.0 ± 0.0 | 1.1 ± 0.2 |
| day 18 | 2.0 ± 1.7 | 6.5 ± 2.4 | 1.0 ± 0.0 | 2.9 ± 1.2 |
| day 26 | 2.5 ± 1.6 | 6.5 ± 2.0 | 1.0 ± 0.0 | 3.2 ± 1.4 |

(mean ± S.D.)

The results obtained after the five times administration at an internal of one week in Test Example 2 (Tables 4-1 to 4-3) were as given below. The IgG antibody titer in the serum in the control group of the oral administration of the aqueous BSA solution was only slightly increased as in the Test Example 1, and no particular difference owing to the administration method was found. On the contrary, in the group of the oral administration of the liposomes in the Examples, the antibody titer was further increased according to the administration schedule of Test Example 2 or, in other words, the antibody titer was evidently higher than that in the control group to which the aqueous BSA solution was given. An increase of the IgA antibody titer in the serum was surely recognized in the liposome-given group, while the IgA antibody titer was unchanged at all even when the dose was increased in the aqueous BSA solution-given group as in Test Example 1. The IgA antibody titer in the saliva in the liposome-given group was far higher than that in the aqueous BSA solution-given group.

Table 4-1    Test Example 2 : Control Example 1 vs. Example 1

Dose of BSA = 50 $\mu$ g/mouse x 5 (day 1, 8, 15, 22 and 29)

| | IgG antibody titer in serum | | IgA antibody titer in serum | |
|---|---|---|---|---|
| | [Control Ex. 1] | [Ex. 1] | [Control Ex. 1] | [Ex. 1] |
| | Aqueous BSA | | Aqueous BSA | |
| | solution | Liposome | solution | Liposome |
| day 1 | 1.0 ± 0.0 | 1.0 ± 0.0 | 1.0 ± 0.0 | 1.0 ± 0.0 |
| day 36 | 1.0 ± 0.0 | 8.4 ± 2.7 | 1.0 ± 0.0 | 1.5 ± 0.5 |
| day 46 | 1.7 ± 0.3 | 8.0 ± 0.0 | 1.0 ± 0.0 | 3.2 ± 1.4 |
| day 55 | 2.0 ± 0.6 | 9.7 ± 1.5 | 1.0 ± 0.0 | 4.2 ± 1.8 |

(mean ± S.D.)

| | IgA antibody titer in saliva | |
|---|---|---|
| | [Control Ex. 1] | [Ex. 1] |
| | Aqueous BSA solution | Liposome |
| day 55 | 0.2± 0.4 | 0.9 ± 0.7 |

(mean ± S.D.)

Table 4-2   Test Example 2 : Control Example 2 vs. Example 2

Dose of BSA = $100\mu$ g/mouse x 5 (day 1, 8, 15, 22 and 29)

| | IgG antibody titer in serum | | IgA antibody titer in serum | |
|---|---|---|---|---|
| | [Control Ex. 2] | [Ex. 2] | [Control Ex. 2] | [Ex. 2] |
| | Aqueous BSA solution | Liposome | Aqueous BSA solution | Liposome |
| day 1 | 1.0 ± 0.0 | 1.0 ± 0.0 | 1.0 ± 0.0 | 1.0 ± 0.0 |
| day 36 | 1.0 ± 0.0 | 5.0 ± 2.9 | 1.0 ± 0.0 | 1.9 ± 1.7 |
| day 46 | 1.0 ± 0.0 | 8.8 ± 1.6 | 1.0 ± 0.0 | 3.2 ± 1.8 |
| day 55 | 2.7 ± 0.8 | 7.8 ± 3.2 | 1.0 ± 0.0 | 4.4 ± 2.1 |

(mean ± S.D.)

| | IgA antibody titer in saliva | |
|---|---|---|
| | [Control Ex. 1] | [Ex. 1] |
| | Aqueous BSA solution | Liposome |
| day 55 | 0.6± 0.8 | 1.3 ± 2.1 |

(mean ± S.D.)

Table 4-3    Test Example 2 : Control Example 4 vs. Example 4

Dose of BSA = 400μ g/mouse x 5 (day 1, 8, 15, 22 and 29)

| | IgG antibody titer in serum | | IgA antibody titer in serum | |
|---|---|---|---|---|
| | [Control Ex. 4] | [Ex. 4] | [Control Ex. 4] | [Ex. 4] |
| | Aqueous BSA solution | Liposome | Aqueous BSA solution | Liposome |
| day 1 | 1.0 ± 0.0 | 1.0 ± 0.0 | 1.0 ± 0.0 | 1.0 ± 0.0 |
| day 36 | 1.7 ± 0.6 | 9.3 ± 2.5 | 1.0 ± 0.0 | 4.0 ± 2.0 |
| day 46 | 2.2 ± 0.9 | 9.7 ± 2.3 | 1.0 ± 0.0 | 5.0 ± 3.2 |
| day 55 | 3.0 ± 1.2 | 9.9 ± 1.7 | 1.0 ± 0.0 | 5.7 ± 2.3 |

(mean ± S.D.)

| | IgA antibody titer in saliva | |
|---|---|---|
| | [Control Ex. 4] | [Ex. 4] |
| | Aqueous BSA solution | Liposome |
| day 55 | 0.1± 0.0 | 1.5 ± 0.8 |

(mean ± S.D.)

It is thus apparent that when the vaccine of the present invention prepared by using bovine serum albumin (BSA) as the model antigens is orally administered, antibodies to the living body is efficiently produced.

Examples 5 to 8

a) Preparation of liposome-type oral vaccine containing influenza HA antigens:

20 ml of liquid chloroform / methanol (9/1) mixture was added to 160 mg of a lipid comprising distearoylphosphatidylcholine (DSPC) / hydrogenated phosphatidylserine (H-PS) / cholesterol (Chol) in a molar ratio of 1/1/2 to dissolve the lipid into the liquid mixture. The solvent was distilled off by heating at 37 °C with a rotary evaporator. After drying in vacuo for two hours, a lipid film was obtained. 4 ml of each of solutions of various concentrations of influenza HA antigens (see Table 5) dispersed in physiological saline buffered with phosphoric acid was added to the lipid film, and the resultant mixture was stirred with a vortex mixer for 10 min to obtain multilamellar liposomes (MLV; multilamellar vesicles). The resultant product was finally diluted to a 1/3.5 concentration with PBS to obtain a liposome-type oral vaccine including influenza HA antigens as shown in the right column in Table 5.

Table 5

| Formulation of liposomes | | | | |
|---|---|---|---|---|
| Ex. | Amount of lipid (mg) | HA Conc. ($\mu$ g/ml) | Final liposome suspension | |
| | | | Lipid conc. ($\mu$ mol/mol) | HA conc. ($\mu$ g/ml) |
| 5 | 160 | 70 | 20 | 20 |
| 6 | 160 | 140 | 20 | 40 |
| 7 | 160 | 280 | 20 | 80 |
| 8 | 160 | 560 | 20 | 160 |
| Final liposome suspension: DSPC/H-PS/Chol = 54.4/51.2/54.4 (mg) | | | | |

Since the dose of the product for each mouse was 0.5 ml, the amount of the lipid given to the mouse was 10$\mu$ mol and that of HA antigens was in the range of 10$\mu$ g (Example 5) to 80 $\mu$ g (Example 8).

b) Immunity test:

The influenza HA antigen-containing liposome suspension thus prepared or a PBS solution of only HA antigens (the dose of HA antigens was made equal to that of the liposomes) was orally administered to each of BALB/c mice (male, 7-weeks old) (each group consisted of 6 mice) in a dose of 0.5 ml with a gastric probe according to a schedule given in Table 6. To determine the antibody titer in the blood and saliva, the blood was taken from the orbits and the saliva was taken by anesthetizing each mouse by intraperitoneal injection of 0.1 ml of 12 mg/ml pentobarbital, administering 0.2 mg/ml pilocarpine by the intraperitoneal injection and taking the saliva from the mouth with a Pasteur pipette.

Table 6

| Immunization schedule | | | | |
|---|---|---|---|---|
| Test | Immunization method | Day of oral administration | Day of taking blood | Day of taking saliva |
| 3 | 5 times at interval of 1 week | 1, 8, 15, 22 and 29 | 36, 46 and 55 | 55 |
| Dose of HA antigens in Test Example 3: 10, 20, 40 and 80 $\mu$ g/time/mouse | | | | |

c) Determination of antibody titer:

IgG and IgA antibody titers in the serum and secretory IgA antibody titer were determined by in the same manner as in the determination of the BSA-containing liposome-type vaccine. It was found that the increase of the antibody titer of the HA antigen-containing liposomes was superior.

Examples 9 to 11

a) Preparation of liposome-type oral vaccine containing influenza virus antigen or hepatitis virus B antigens:

50 ml of liquid chloroform / methanol (9/1) mixture was added to a mixture of 19.3 mg of distearoyl phosphatidyl choline (DSPC), 19.3 mg of cholesterol (Chol) and 18.2 mg of hydrogenated soybean phosphatidyl serine (HSPS) to dissolve the mixture into the liquid mixture. The solvent was distilled off by heating at 50°C with a rotary evaporator. After drying in vacuo for two hours, a lipid film was obtained. 2 ml of a suspension of antigens (Table 7) in physiological saline buffered with phosphoric acid (PBS) was added to the lipid film, and the resultant mixture was stirred with a vortex mixer at 50°C for 10 min. The resultant mixture was filtered through a polycarbonate membrane filter having a pore diameter of 0.6$\mu$ m under heating at 50 °C twice to obtain multilamellar liposomes.

Table 7

| Ex. 9 | Inactivated influenza virus antigens | A/PR/8(HINI) strain | 2 mg/ml |
|---|---|---|---|
| Ex. 10 | Influenza virus HA antigens | A/PR/8(HINI) strain | 2 mg/ml |
| Ex. 11 | Hepatitis virus B antigens | yeast (gene recombination) | 0.4 mg/ml |

The particle diameter of the liposome-type oral vaccines thus obtained were determined by a quasi-elastic light scattering method (Dynamic light scattering meter DLS-700; a product of Otsuka Denshi Co., Ltd.). The results are given in Table 8. Liposomes prepared in the absence of the antigen protein were used as the control.

Table 8

| Weight-average particle diameter ± standard deviation (mn) | |
|---|---|
| Ex. 9 | 408.2 ± 198 |
| Ex. 10 | 482.5 ± 213 |
| Ex. 11 | 443.1 ± 201 |
| Control Ex. | 421.6 ± 206 |

The form of these oral vaccines was examined by staining them by a negative staining method and observing them with a transmission electron microscope. It was confirmed that they were in the form of liposomes in the Examples and Control Example.

Oral vaccines containing a phosphatidylserine selected from the group consisting of hydrogenated egg yolk phosphatidylserine, dimyristoylphosphatidylserine, dipalmitoylphosphatidylserine, distearoylphosphatidylserine and phosphatidylserine derived from the bovine brain can be prepared in the same manner as that described above.

Example 12

Multilamellar liposomes were prepared by using 6.4 mg of 4-O-(6-0-eicosanoyl- $\beta$ -D-mannopyranosyl)-D-mannopyranose, 32.0 mg of distearoylphosphatidylcholine, 19.3 mg of cholesterol, 5.5 mg of dicetyl phosphate and 2 ml of inactivated influenza virus antigens (2 mg/ml) derived from A/PR/8(HINI) in the same manner as that of Example 9.

Example 13

Preparation of oral vaccine containing mannan derivative as component of membrane:

Multilamellar liposomes were prepared by using 32.0 mg of distearoylphosphatidylcholine, 19.3 mg of cholesterol, 5.5 mg of dicetyl phosphate and 2 ml of inactivated influenza virus antigens (2 mg/ml) derived from A/PR/8(HINI) in the same manner as that of Example 9.

10.0 mg of N-(2-N'-cholesterylcarboxyaminomethyl) carboxymethylmannan was dissolved in 10 ml of PBS, and the resultant solution was added to a suspension of the above-described liposomes. The mixture thus obtained was stirred for 2 hours while the liquid temperature was kept at 20 °C to obtain liposome-type oral vaccine containing the mannan derivative as a component of the membrane.

The particle diameter of the oral vaccines obtained in Examples 12 and 13 was determined in the same manner as that described above. The weight average particle diameter± standard deviation (nm) was 443.7 ± 208 (nm) in Example 12 and 450.7 ± 211 (nm) in Example 13. It was confirmed by the observation with the transmission electron microscope in the same manner as above that the products were in the form of the liposomes.

Oral vaccines containing a mannose derivative selected from mannose residue-containing phosphatidylethanolamine derivatives, mannose residue-containing cholesterol derivatives, dimannosyl diglycerides and phosphatidylmannoses can be prepared in the same manner as that described above.

Example 14

Preparation of oral vaccine containing mannose derivative or mannan derivative and phosphatidyl serine as components of membrane:

Multilamellar liposomes were prepared by using 20.0 mg of hydrogenated soybean phosphatidylserine, 20.0 mg of distearoylphosphatidylcholine and 19.3 mg of cholesterol in the same manner as that of Example 9.

10.0 mg of N-(2-N'-cholesterylcarboxymethyl)carbamylmethylmannan was dissolved in 10 ml of PBS, and the resultant solution was added to a suspension of the above-described liposomes. The mixture thus obtained was stirred for 2 hours while the liquid temperature was kept at 20 °C to obtain the oral vaccine.

The particle diameter of the oral vaccine thus obtained had a weight average particle diameter ± standard deviation of 476.9± 206 (nm). It was confirmed by the observation with the transmission electron microscope that the product was in the form of the liposomes.

Example 15

Preparation of oral vaccine containing adjuvant:

Multilamellar liposomes were prepared by using 10.0 mg of [6-0-(2-tetradecylhexadecanoyl)-N-acetylmuramoyl)-L-alanyl-D-glutamine, 20.0 g of hydrogenated soybean phosphatidylserine, 20.0 mg of distearoylphosphatidylcholine and 19.3 mg of cholesterol in the same manner as that of Example 9. The weight average particle diameter ± standard deviation of the product was 461.2± 210 (nm). It was confirmed by the observation with the transmission electron microscope that the product was in the form of the liposomes.

Oral vaccines containing an adjuvant selected from [6-0-(2-tetradecylhexadecanoyl)-N-acetylmuramoyl]-L-alanyl-D-glutamamide and $N^2$-(N-acetylmuramoyl-L-alanyl-D-isoglutaminyl)-$N^6$-stearoyl-L-lysine can be prepared in the same manner as that described above.

b) Immunity test:

The liposome suspension containing inactivated influenza virus antigens (inactivated whole virus particles immobilized with 0.05 % formalin) obtained in Example 9 was orally administered to each of BALB/cCr Slc mice (male, 7-weeks old) (each group consisted of 5 mice) in a dose of 0.2 ml with a gastric prove for mice according to a schedule given in Table 9. Three weeks after the initiation of the administration (on the 21st or 22nd day), the serum and intestinal tract-wash solution were taken from the mice of each group, and the IgG and IgA antibody titers specific to the given antigens were determined by the ELISA method.

The serum and intestinal tract-wash solution were obtained by the following processes: the axillary vein of each mouse was cut off under anesthesia with ether to collect the blood. The blood was centrifuged at 3,000 rpm for 10 minutes to separate the serum to be used for the determination of the antibodies. After the completion of the blood collection, the abdomen of the mouse was incised, and the intestines thereof was taken out. The intestines was cut at a portion between the ileum and the cecum. 5 ml of a sampling solution (ELISA mate; a diluent/blocking solution of KPL Co.) was slowly injected into a portion between the stomach and the duodenum with a syringe (10 ml) having 25 needles, and the intestinal tract-wash solution discharged from the end of the ileum was received in a Petri dish. The wash solution was centrifuged at 2,000 rpm for 10 min, and the antibodies in the supernatant liquid were determined. The samples thus obtained were kept at -20 °C until the determination.

14

Table 9

| Immunization schedule | | | |
|---|---|---|---|
| Test | Immunization method | Day of oral administration | Day of sampling |
| 1 | Continuous administration for 5 days | 1, 2, 3, 4 and 5 | 21 |
| 2 | 5 times at interval of 4 days | 0, 4, 8, 12 and 16 | 21 |
| 3 | Continuous administration for 5 days followed by 3 times administration at interval of 4 days | 0, 1, 2, 3, 4, 8, 12 and 16 | 21 |
| Dose of antigens: 400μ g/time/mouse | | | |

c) Determination of antibody titer:

IgG and IgA antibody titers specific to the administered antigens in the serum and the intestinal tract-wash water were determined with an ELISA determination kit (ELISA mate) of KPL (Kirlegaard & Perry Lab. Inc.) by a method described in the protocol thereof.

The production of IgA and IgG specific to the antigens was recognized in the serum and the the production of IgA was recognized in the intestinal tract-wash water.

[ELISA method]

50μ l of 2.5μ g/ml antigens dissolved in a coating buffer solution was fed into each of 96 wells of a microplate and left to stand at room temperature for 1 hour to immobilize the antigens on the microplate. The solution in each well was discharged, and the plate was patted on a paper towel to completely remove the solution. 100μ l of a blocking solution was fed into each well and the plate was left to stand at room temperature for 5 min. After removing the solution from the well, 50 μ l/well of each of diluted solution (diluted two times, four times, eight times or the like) of the serum and the intestinal tract-wash solution was fed into the well. After leaving the plate to stand at room temperature for 1 hour, the solution was removed from each well. After washing with 200 to 300μ l of the washing water four times, the well was filled with the washing water and then left to stand at room temperature for 5 minutes. After removing the water from the well, 50 μ l of the peroxidase substrate solution was fed into each well and then the plate was left to stand at room temperature for 20 min. 50μ l of a peroxidase reaction-terminating solution was fed into each well to terminate the reaction. The reaction mixture was mixed with a plate mixer. The absorbance at 405 nm in each well was determined with ELISA READER (Multiskan PLUS: Titertek Co.). The antibody titer was determined as follows: The absorbance of a standard sample (i.e. a corresponding sample having a known antibody titer) at each dilution rate was put in the following ligit-log transformation formula 1 to find the value Y. The logit Y was plotted as the ordinates and the logarithmic values of the antibody titer was plotted as the abscissae. A straight standard line was drawn, from which the antibody titer of each sample was calculated.

logit-log transformation formula:

$Y = a + bX$  $Y = $ logit $y = \log(y/(1-y))$: $y = (ODx-ODb)/(ODm-PDb)$
$X = \log 2X$ : $x = $ antibody titer

ODx:    absorbance at each dilution (antibody titer)
ODm:    the maximum absorbance
ODb:    absorbance of the blanc

**Claims**

1. An oral vaccine comprising a complex of antigens and a lipid, the lipid comprising a glycolipid having mannose and/or a phospholipid which is a phosphatidylserine.

2. The oral vaccine according to Claim 1, wherein the complex is a lipid membrane structure.

3. The oral vaccine according to Claim 2, wherein the lipid membrane structure is a liposome.

4. The oral vaccine according to Claim 2, wherein the lipid membrane structure is an emulsion.

5. The oral vaccine according to Claim 2, wherein the lipid membrane structure is a micelle.

6. The oral vaccine according to any of Claims 1 to 5, wherein the glycolipid having mannose is 4-0-(6-0-eicosanoyl-$\beta$-D-mannopyranosyl)-D-mannopyranose or N-(2-N'-cholesterylcarboxymethyl) carbamylmethylmannan.

7. The oral vaccine according to Claim 1, which further contains, as a component of the membrane, a lipid other than the glycolipid having mannose and/or the phospholipid which is a phosphatidylserine.

8. The oral vaccine according to Claim 1, which further contains, as a component of the membrane, a phosphatidylcholine selected from a phosphatidylcholine derived from soybeans or egg yolks, hydrogenated phosphatidylcholine, dimyristoylphosphatidylcholine, dipalmitoylphosphatidylcholine and distearoylphosphatidylcholine, and/or a charged lipid and/or a sphingomyelin in addition to the glycolipid having mannose and/or the phospholipid which is a phosphatidylserine.

9. The oral vaccine according to Claim 1, which further contains, as a component of the membrane, a charged lipid in addition to the glycolipid having mannose and/or the phospholipid which is a phosphatidylserine.

10. The oral vaccine according to Claim 9, wherein the charged lipid is a dialkyl phosphate, diacylphoaphatidic acid, stearylamine, phosphatidylglycerol, phosphatidylinositol, phosphatidylethanolamine or ionic surfactant.

11. The oral vaccine according to any of Claims 1 to 5, wherein the lipid is a phosphatidylcholine derived from soybeans, phosphatidylcholine derived from egg yolks, hydrogenated phosphatidylcholine, distearoylphosphatidylcholine or dipalmitoylphosphatidylcholine and a phosphatidylserine and, if necessary, a cholesterol.

12. The oral vaccine according to Claim 1, wherein the antigens are microbial antigens or attenuated microorganisms.

13. The oral vaccine according to Claim 1, wherein the antigens are influenza virus antigens, hepatitis virus A antigens, hepatitis virus B antigens or hepatitis virus C antigens.

14. The oral vaccine according to Claim 1, wherein the antigens are influenza hemaglutinine.

15. The oral vaccine according to Claim 1, which contains an adjuvant in addition to the antigens and the lipid.

16. The oral vaccine according to Claim 15, wherein the adjuvant is a muramyl dipeptide (MDP) derivative.

17. The oral vaccine according to Claim 16, wherein the adjuvant is (N-acetylmuramoyl)-L-alanyl-D-isoglutamine, 6-0-(2-tetradecylhexadecanoyl)-N-acetylmuramoyl)-L-alanyl-D-isoglutamine, 6-0-(2-tetradecylhexadecanoyl)-N-acetylmuramoyl)-L-alanyl-D-glutamamide or $N^2$-[(N-acetylmuramoyl)-L-alanyl-D-isoglutaminyl]-$N^6$-stearoyl-L-lysine.

18. A method for improving the immunity of an individual by orally administrating an oral vaccine comprising a complex of antigens and a lipid, the lipid comprising a glycolipid having mannose and/or a phospholipid which is a phosphatidylserine.

19. The method according to Claim 18, wherein the complex is a lipid membrane structure.

20. The method according to Claim 18, wherein the lipid membrane structure is a liposome.

**21.** The method according to Claim 18, wherein the lipid membrane structure is an emulsion.

**22.** The method according to Claim 18, wherein the lipid membrane structure is a micelle.

**23.** The method according to any of Claims 18 to 22, wherein the glycolipid having mannose is 4-0-(6-0-eicosanoyl- $\beta$ -D-mannopyranosyl)-D-mannopyranose or N-(2-N'-cholesterylcarboxymethyl) carbamylmethylmannan.

**24.** The method according to Claim 18, which further contains, as a component of the membrane, a lipid other than the glycolipid having mannose and/or the phospholipid which is a phosphatidylserine.

**25.** The method according to Claim 24, wherein the other lipid is a phosphatidylcholine selected from a phosphatidylcholine derived from soybeans, phosphatidylcholine derived from egg yolks, hydrogenated phosphatidylcholine, dimyristoylphosphatidylcholine, dipalmitoylphosphatidylcholine and distearoylphosphatidylcholine, and/or a charged lipid and/or a sphingomyelin.

**26.** The method according to Claim 24, wherein the vaccine further contains, as a component of the membrane, a charged lipid in addition to the glycolipid having mannose and/or the phospholipid which is a phosphatidylserine.

**27.** The method according to Claim 26, wherein the charged lipid is a dialkyl phosphate, diacylphoaphatidic acid, stearylamine, phosphatidylglycerol, phosphatidylinositol, phosphatidylethanolamine or ionic surfactant.

**28.** The method according to Claim 18, wherein the lipid is a phosphatidylcholine derived from soybeans, phosphatidylcholine derived from egg yolks, hydrogenated phosphatidylcholine, distearoylphosphatidylcholine or dipalmitoylphosphatidylcholine and a phosphatidylserine and, if necessary, a cholesterol.

**29.** The method according to Claim 18, wherein the antigens are microbial antigens or attenuated microorganisms.

**30.** The method according to Claim 18, wherein the antigens are influenza virus antigens, hepatitis virus A antigens, hepatitis virus B antigens or hepatitis virus C antigens.

**31.** The method according to Claim 18, wherein the antigens are influenza hemaglutinine.

**32.** The method according to Claim 18, which contains an adjuvant in addition to the antigens and the lipid.

**33.** The method according to Claim 32, wherein the adjuvant is a muramyl dipeptide (MDP) derivative.

**34.** The method according to Claim 18, wherein the vaccine contains (N-acetylmuramoyl)-L-alanyl-D-isoglutamine, 6-0-(2-tetr adecylhexadecanoyl)-N-acetylmuramoyl)-L-alanyl-D-isoglutamine, 6-0-(2-tetradecylhexadecanoyl)-N-acetylmuramoyl)-L-alanyl-D-glutamamide or $N^2$-[(N-acetylmuramoyl)-L-alanyl-D-isoglutaminyl]-$N^6$-stearoyl-L-lysine as the adjuvant in addition to the antigens and lipids.

International application No.

PCT/JP93/00264

## A. CLASSIFICATION OF SUBJECT MATTER

Int. Cl$^5$  A61K39/00

According to International Patent Classification (IPC) or to both national classification and IPC

## B. FIELDS SEARCHED

Minimum documentation searched (classification system followed by classification symbols)

Int. Cl$^5$  A61K39/00, A61K9/107, A61K9/127

Documentation searched other than minimum documentation to the extent that such documents are included in the fields searched

Electronic data base consulted during the international search (name of data base and, where practicable, search terms used)

## C. DOCUMENTS CONSIDERED TO BE RELEVANT

| Category* | Citation of document, with indication, where appropriate, of the relevant passages | Relevant to claim No. |
|---|---|---|
| A | JP, B1, 41-5475 (Glakuso Laboratories Ltd.), March 26, 1966 (26. 03. 66), & DE, B, 1467907 & FR, A, 1546452 & US, A, 3376199 | 1-17 |
| Y | JP, A, 63-107742 (Wako Pure Chemical Industries, Ltd.), March 12, 1988 (12. 05. 88), & EP, A, 247497 & US, A, 4861597 | 1-10, 12-17 |
| Y | JP, A, 52-44228 (The Wellcome Foundation Ltd.), April 7, 1977 (07. 04. 77), & DE, A, 2643641 & FR, A, 2325387 & US, A, 4196191 | 1-5, 7-17 |
| Y | WO, A, 89/08499 (Nippon Seika K.K.), September 21, 1989 (21. 09. 89), & US, A, 5169636 | 1-10, 12-17 |
| Y | JP, A, 1-197431 (Lederle Japan, Ltd.), August 9, 1989 (09. 08. 89), | 1-10, 12-17 |

[X] Further documents are listed in the continuation of Box C.  [ ] See patent family annex.

* Special categories of cited documents:

"A" document defining the general state of the art which is not considered to be of particular relevance

"E" earlier document but published on or after the international filing date

"L" document which may throw doubts on priority claim(s) or which is cited to establish the publication date of another citation or other special reason (as specified)

"O" document referring to an oral disclosure, use, exhibition or other means

"P" document published prior to the international filing date but later than the priority date claimed

"T" later document published after the international filing date or priority date and not in conflict with the application but cited to understand the principle or theory underlying the invention

"X" document of particular relevance; the claimed invention cannot be considered novel or cannot be considered to involve an inventive step when the document is taken alone

"Y" document of particular relevance; the claimed invention cannot be considered to involve an inventive step when the document is combined with one or more other such documents, such combination being obvious to a person skilled in the art

"&" document member of the same patent family

| Date of the actual completion of the international search | Date of mailing of the international search report |
|---|---|
| May 13, 1993 (13. 05. 93) | June 1, 1993 (01. 06. 93) |

| Name and mailing address of the ISA/ | Authorized officer |
|---|---|
| Japanese Patent Office | |
| Facsimile No. | Telephone No. |

Form PCT/ISA/210 (second sheet) (July 1992)

| | International application No. |
| --- | --- |
| | PCT/JP93/00264 |

**C (Continuation).  DOCUMENTS CONSIDERED TO BE RELEVANT**

| Category* | Citation of document, with indication, where appropriate, of the relevant passages | Relevant to claim No. |
| --- | --- | --- |
| | (Family: none) | |
| Y | JP, A, 62-294432 (Centre National de La Recherche Scientifique (C.N.R.S.)) December 21, 1987 (21. 12. 87), & EP, A, 241376 & FR, A, 2596651 | 1-10, 12-17 |
| Y | JP, A, 62-30708 (Takeda Chemical Industries, Ltd.), February 9, 1987 (09. 02. 87), & US, A, 4883665 | 1-10, 12-17 |
| Y | JP, A, 55-130920 (Merck & Co., Inc.), October 11, 1980 (11. 10. 80), & US, A, 4199565 & EP, A, 17557 | 10 |
| Y | New Formulation Development System General Technology-Base·Additive-issued by R & D Planning, July 12, 1985 (12. 07. 85), P. 239, 240, 245, 246, 267 | 1-17 |